# FASCICULE DE BREVET EUROPEEN

(11) **EP 0 698 034 B1**
(45) Date de publication et mention de la délivrance du brevet: **06.08.1997**
(21) Numéro de dépôt: 94916273.9
(22) Date de dépôt: 11.05.1994
(51) Int. Cl.: C07H 21/00

(54) **OLIGONUCLEOTIDES PHOSPHOROTHIOATES TRIESTERS ET PROCEDE DE PREPARATION**
TRIESTERS VON PHOSPHOROTHIOATEN OLIGONUKLEOTIDEN UND VERFAHREN ZUR HERSTELLUNG
TRIESTER PHOSPHOROTHIOATE OLIGONUCLEOTIDES AND METHOD OF PREPARATION

(30) Priorité: 12.05.1993 FR 9305706
(43) Date de publication de la demande: 28.02.1996
(73) Titulaire: CENTRE NATIONAL DE LA RECHERCHE SCIENTIFIQUE (CNRS), 75794 Paris Cédex 16 (FR)
(72) Inventeur: IMBACH, Jean-Louis, F-34000 Montpellier (FR); RAYNER, Bernard, F-34100 Montpellier (FR)
(74) Mandataire: Warcoin, Jacques
(86) Numéro de dépôt international: FR9400563
(87) Numéro de publication internationale: WO9426764

(56) Documents cités:
- EP-A- 0 463 712
- EP-A- 0 519 463
- WO-A-91/04983
- US-A- 5 210 264

## Description

La présente invention concerne des composés oligonucléotides phosphorothioates triesters et un procédé de préparation.

Dans la présente demande, le terme "oligonucléotides" désigne d'une façon générale un polynucléotide d'ADN ou d'ARN, c'est-à-dire en série ribo- (ARN) ou désoxyribo- (ADN), ou encore mixte ribo-désoxyribo. Ces oligonucléotides sont constitués en général d'un enchaînement de 2 à 100, et plus généralement, de 5 à 50 nucléotides.

Les oligonucléotides sont utilisés à des fins biologiques ou thérapeutiques selon différentes approches : antisens (formation de duplex), anti-gène (formation de triples hélices), catalytique (ARN à activité ribozymique) ou sens (protéine cible).

Les oligonucléotides antisens sont de courtes molécules synthétiques d'ADN -ou d'ARN- ou mixtes, de séquences complémentaires à une séquence cible appartenant à un gène ou à un ARN messager dont on désire bloquer spécifiquement l'expression. Les oligonucléotides antisens sont en effet dirigés contre une séquence d'ARN messager, ou bien contre une séquence d'ADN et s'hybrident à la séquence dont ils sont complémentaires, pouvant ainsi bloquer l'expression génétique.

Les désoxyribonucléotides antisens peuvent également être dirigés contre certaines régions d'ADN bicaténaire (séquences homopurines/homopyrimidines ou riches en purines/pyrimidines) et former ainsi des triples hélices. Ces oligonucléotides dirigés ainsi contre l'ADN ont été appelés "anti-gène" ou encore "anti-code". La formation d'une triple hélice, au niveau d'une séquence particulière, peut bloquer la fixation de protéines intervenant dans l'expression d'un gène et/ou permettre d'introduire de dommages irréversibles dans l'ADN si l'oligonucléotide considéré possède un groupement réactif particulier. De tels oligonucléotides antisens peuvent constituer des endonucléases de restriction artificielles, dirigées contre des séquences spécifiques.

Dans les cellules, et plus particulièrement dans un organisme, dans la circulation sanguine par exemple, les oligonucléotides naturels sont sensibles à la dégradation par les nucléases. Les nucléases sont des enzymes de dégradation capables de couper les liaisons phosphodiester de l'ADN ou de l'ARN, soit en introduisant des coupures internes sur des molécules mono- ou bicaténaires, soit en attaquant ces molécules à partir de leurs extrémités. Les enzymes qui attaquent de façon interne sont appelées les endonucléases et celle attaquant par les extrémités sont appelées exonucléases.

L'utilisation des oligonucléotides se heurte à deux problèmes majeurs qui sont d'une part, une grande sensibilité à la dégradation par des exonucléases, qui se trouvent aussi bien dans le sérum ou en milieu extra-cellulaire ou en milieu cytoplasmique intracellulaire et, d'autre part, une faible pénétration intracellulaire.

L'utilisation d'oligonucléotides modifiés a déjà été proposée pour augmenter la stabilité des oligonucléotides ou favoriser la pénétration à travers les membranes cellulaires ou encore pour stabiliser l'hybridation et l'affinité spécifique pour une séquence cible, que ce soit un acide nucléique mono ou double brin, ou même une protéine, ou encore pour accroître l'interaction avec la dite séquence cible. On a proposé des modifications chimiques du squelette structurel de la molécule ou des dérivations ou couplages à des groupements réactifs ou intercalants, en général localisés à l'extrémité des oligonucléotides.

En ce qui concerne les modifications chimiques du squelette, on a proposé de modifier la nature de l'enchaînement phosphate internucléotidique, notamment sous forme de méthyl-phosphonate, phosphorothioate ou phosphorodithioate ; ou encore en modifiant la partie sucre, notamment par une configuration anomérique alpha, une substitution 2'-O-CH₃ ou en remplaçant l'oxygène du cycle furanosique par un soufre (4'-thio-ribonucléotide). On a également proposé de modifier les bases de nucléotides.

Ainsi, dans les demandes de brevet français FR 83 01223 (2 540 122) et FR 84 11795 (2 568 254) ont été décrits des composés chimiques constitués par un oligonucléotide comportant un enchaînement de β-nucléotides naturels ou modifiés, sur lesquels se trouve fixé par une liaison covalente au moins un groupe intercalant, qui possèdent la propriété de bloquer sélectivement l'expression d'un gène et qui, de ce fait, sont particulièrement utiles en thérapeutique comme substances antivirales, antibiotiques, antiparasitaires ou antitumorales.

Dans la demande internationale WO 88/04301 ont été décrits des oligonucléotides de configuration anomérique alpha présentant des appariements parallèles plus stables avec des séquences complémentaires.

Toutefois, les utilisations proposées jusqu'à maintenant soulèvent d'autres problèmes, notamment de toxicité, des modifications chimiques introduites dans la molécule pouvant se révéler induire des toxicités au niveau pharmacologique dans certaines applications thérapeutiques. D'une manière générale, il est difficile de combiner les différents critères de résistance aux nucléases, stabilisation de l'hybridation, pénétration dans la cellule et, également, accroissement de l'activité en rendant le duplex avec la séquence cible complémentaire substrat de la RNAse H, laquelle a la propriété de cliver les duplex ADN/ARN.

Les composés oligonucléotides dont la partie phosphate était modifiée en méthyl-phosphonate sont particulièrement résistants à la dégradation par les nucléases. Par ailleurs, les oligomères électriquement neutres comme les oligomères de type méthylphosphonate pénètrent plus facilement dans la cellule que les oligomères électriquement chargés comme les phosphodiesters. Toutefois, ces dérivés méthylphosphonates présentent une chiralité, notamment au niveau du phosphate, conduisant ainsi à la formation de diastéréoisomères. En outre, les duplex ARN/oligodésoxynucléotides méthyl-phosphonate ne sont pas substrats de la RNAseH. Les dérivés oligomères du type phosphorothioates diesters présentent une résistance à la dégradation par les nucléases mais à un degré moindre que les oligomères méthylphosphonates. Ils conduisent en revanche à des oligomères électriquement chargés capables d'activer la RNase H mais pénètrent moins facilement dans la cellule que les oligomères méthylphosphonates.

D'une façon générale, les oligonucléotides faisant l'objet de l'invention sont destinés à fournir de nouveaux oligonucléotides stables capables d'être internalisés dans les cellules et capables de s'hybrider et/ou de présenter une affinité pour des séquences spécifiques d'acides nucléiques ou de protéine et donc d'interagir avec des facteurs cellulaires ou viraux.

La présente invention fournit des oligonucléotides à enchaînements phosphorothioates triesters électriquement neutres pouvant redonner, après pénétration dans la cellule, et sélectivement, des liaisons phosphorothioates diesters ou phosphodiesters polaires. D'une façon analogue, la présente invention inclut des enchaînements phosphorodithioates triesters délivrant intracellulairement des liaisons phosphorodithioates diesters ou phosphorothioates diesters.

Pour ce faire, on protège sélectivement des liaisons P-S⁻ d'un oligomère par un groupement (X) bioréversible dans les milieux intracellulaires.

Les modifications proposées selon l'invention fournissent des oligomères ayant les propriétés avantageuses des dérivés phosphorothioates diesters, tout en palliant aux inconvénients de ces derniers, notamment en ce qui concerne leur sensibilité aux exonucléases extracellulaires et leur difficulté de pénétration à travers la membrane cellulaire.

La présente invention a donc plus précisément pour objet un oligonucléotide phosphorothioate ou phosphorodithioate triester caractérisé en ce qu'il comporte des enchaînements internucléotidiques qui présentent une liaison P-S⁻ protégée par un groupement (X) bioréversible dans les milieux intracellulaires.

La voie de synthèse utilisée dans cette invention consiste en l'utilisation d'une réaction de substitution nucléophile par l'atome de soufre de la liaison P-S⁻ d'un agent alkylant XL, L étant un groupement partant (halogène, ester, tosyl...) et X un groupement bioréversible. Il s'ensuit qu'il est possible de transformer les fonctions P-S⁻ en phosphotriesters bioréversibles correspondants, comme il sera montré dans les exemples ci-après.

Un tel procédé ne nécessite pas une protection des bases hétérocycliques et peut donc être effectué directement sur un oligonucléotide préalablement préparé. On peut ainsi parvenir à des oligomères phosphorothioates triesters à partir d'oligomères phosphorothioates diesters. En effet, un enchaînement mixte comprenant des liaisons phosphodiesters (P-O⁻) et phosphorothioates (P-S⁻) sera sélectivement alkylé au niveau des atomes de soufre.

Les oligomères phosphorothioates triesters selon la présente invention sont électriquement neutres dans le milieu extracellulaire et bénéficient de ce fait d'une pénétration améliorée dans la cellule. En outre, ils permettent de délivrer intracellulairement des oligonucléotides phosphodiesters ou phosphorothioates diesters électriquement chargés susceptibles, à ce titre, d'être substrats de la RNAse H lorsqu'ils forment un duplex mixte ARN/ADN avec leurs séquences complémentaires cibles.

Le principe de l'invention s'applique à tout oligonucléotide de synthèse, de série ADN, ARN ou mixte, quelle que soit la cible biologique envisagée, dans la mesure où il présente des enchaînements internucléotidiques comportant une liaison P-S⁻ susceptible d'être alkylée par des groupements bioréversibles.

Dans un mode de réalisation, les oligonucléotides selon la présente invention répondent à la formule 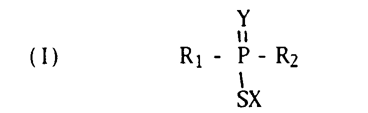 dans laquelle :
- Y représente O ou S
- R₁ et R₂ sont respectivement un résidu en 3'-O et 5'-O d'un nucléoside ou d'un oligonucléotide dont l'enchaînement internucléotidique est naturel ou modifié, notamment dont l'enchaînement est du type phosphorothioate triester de formule 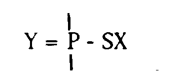
- X est un radical -(CH₂)ₙ-Y¹ - W où
   . Y¹ représente S ou O
   . n varie de 1 à 6
   . W représente :
      - soit SU avec U qui représente un radical alkyle, aryle, ou osidique éventuellement substitués,
      - soit 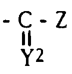 avec Y² qui représente O ou S, et
         Z qui représente un radical alkyle, aryle, ou osidique, éventuellement substitués.

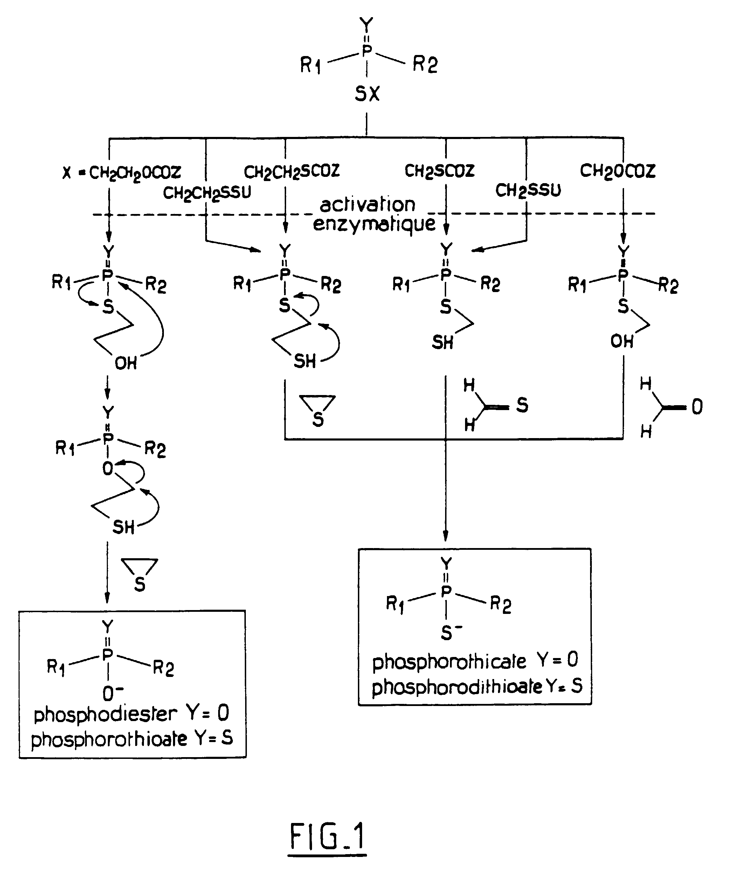

On entend ici par "oligonucléotide modifié" des modifications dans la partie sucre, dans la base ou encore dans l'enchaînement phosphate, voire dans la configuration anomérique des enchaînements. Ces groupements X subissent une coupure enzymatique de la liaison Y₁ 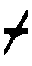 W par l'activation enzymatique d'enzymes intracellulaires selon un mécanisme illustré dans la Figure 1.

Lorsque U et Z représentent un radical alkyle, aryle ou osidique, on cite en particulier les radicaux alkyle en C₁ et C₇, benzyle, phényl, et comme sucres le glucose, mannose ou rhamnose.

Parmi ces groupements X, on cite plus particulièrement -(CH₂)ₙ - S-S - U ou 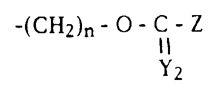 et plus particulièrement encore 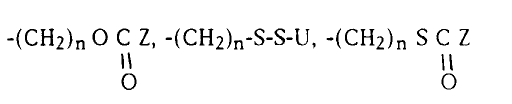 avec n = 1 ou 2.

Ces enchaînements phosphorothioates triesters sont transformés sous l'influence d'enzymes intracellulaires, soit en phosphodiesters, soit en phosphorothioates diesters, comme il est montré par les études de décomposition en extraits cellulaires présentées ci-après et les mécanismes indiqués dans la Figure 1.

Parmi les alkyles constituant les groupes U ou Z, on cite en particulier les alkyles inférieurs éventuellement substitués par un groupe choisi parmi hydroxy, SH ou NH₂.

Ainsi, dans un mode de réalisation particulier de l'invention, X représente -(CH ₂)ₙ - S - S - (CH₂)_{n¹} - X¹ avec n et n¹ qui représentent un entier de 1 à 4, de préférence 2 et X¹ représente H, OH, SH ou NH₂, ou X représente -(CH₂)ₙ - Y¹ - CO - Z, avec n = 1 ou 2 et Z = CH₃ ou tBu. Plus particulièrement encore X = tBu COOCH₂-, CH₃ COSHCH₂CH₂-, ou CH₃COSCH₂-.

Les oligomères phosphorothioates diesters électriquement chargés sont quelque peu sensibles à la dégradation nucléasique intracellulaire. C'est pourquoi, dans un mode de réalisation avantageux selon l'invention, les oligonucléotides seront constitués d'un oligomère chimérique comportant une séquence centrale d'ADN ou d'ARN dont les enchaînements internucléotidiques comportent une liaison 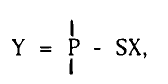 cette séquence centrale étant flanquée en 5' et 3' de séquences d'ADN ou ARN modifiées de sorte qu'elles résistent aux nucléases et/ou stabilisent l'hybridation avec un brin complémentaire.

Ce mode de réalisation est particulièrement avantageux dans le cas d'une approche anti-sens dans laquelle on utilise un oligonucléotide de type ADN, de façon à former un duplex ADN/ARN qui sera ainsi substrat de la RNAse H. Ces oligomères chimériques selon l'invention combinent toutes les propriétés avantageuses de pénétration cellulaire, résistance aux enzymes intra- et extracellulaires et formation de duplex substrats de la RNAse H.

En particulier, on utilisera des composés de formule I dans laquelle R₁ et R₂ ont des séquences aux extrémités 5' et 3' avec des enchaînements internucléotiques, électriquement neutres, qui résistent à la dégradation par les nucléases extra- et intra cellulaires, tels que des enchaînements du type méthylphosphonate.

Selon un autre mode de réalisation, les oligonucléotides selon l'invention sont constitués d'un oligomère chimérique comportant une séquence centrale d'ADN ou d'ARN d'anomérie β, dont les enchaînements internucléotidique s sont du type Y = P - SX, cette séquence centrale étant flanquée en 5' et 3' de séquences d'ADN ou d'ARN d'anomérie alpha.

En particulier, un oligonucléotide selon l'invention peut être constitué d'un oligomère chimérique contenant une séquence centrale d'enchaînement β-nucléosides phosphorothioates triesters à enchaînement internucléotidique de type 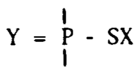 encadrée par des flancs constitués de séquences d'enchaînements d'alpha nucléosides phosphates diesters.

D'une manière générale, différents nucléotides peuvent rentrer dans la formulation d'oligonucléotides selon l'invention. Les oligonucléotides faisant l'objet de la présente invention peuvent être composés par une séquence de bases nucléotidiques comportant de l'adénine (A), de la guanine (G), de la cytosine (C), de la thymine (T) et de l'uracile (U), ou les oligonucléotides peuvent également comporter des nucléotides rares (Inosine, I, ou rI par exemple) ou des nucléotides modifiés, soit en série désoxyribo-, soit en série ribo-, reliés entre elles par des liaisons phosphodiesters non-modifiées ou modifiées selon la présente invention.

En particulier, les oligonucléotides peuvent comporter des nucléotides réactifs, capables d'établir des liens avec la séquence de la molécule cible complémentaire à l'oligonucléotide.

Ainsi, les oligonucléotides selon l'invention peuvent porter des groupements réactifs greffés sur les nucléotides, comme par exemple des groupements psoralène, ou d'autres agents de pontage ou agents intercalant pouvant réagir avec la séquence de la molécule cible complémentaire à l'oligonucléotide.

Font également partie de l'invention des oligonucléotides couplés à des molécules permettant d'accroître leur pénétration intracellulaire, et en particulier des groupements lipophiles, des polypeptides ou des protéines.

Les oligonucléotides selon l'invention sont préparés à partir d'un oligonucléotide présentant des enchaînements internucléotidiques phosphothioates diesters du type 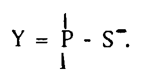 On effectue une réaction de substitution nucléophile par l'atome de soufre de la liaison -P-S⁻ des dits enchaînements phosphorothioates diesters sur un agent alkylant XL conduisant ainsi à la formation d'un oligonucléotide d'enchaînements phosphorothioates triesters 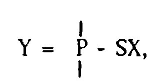 L étant un groupe partant tel que halogène, ester, tosyl, et X étant un groupement bioréversible selon l'invention.

Les oligonucléotides phosphorothioates diesters à enchaînement 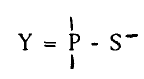 sont préparés par les méthodes conventionnelles de synthèse, par voie chimique, biochimique, ou par des approches faisant appel à des combinaisons des techniques de la chimie de synthèse et de la biologie moléculaire.

Parmi les méthodes qualifiées de conventionnelles, diverses méthodes de synthèse chimique d'oligonucléotides ont été développées et sont bien connues des spécialistes travaillant dans ces domaines. Par exemple, une méthode consiste à utiliser un support solide dit CPG (controlled pore glass) sur lequel le premier nucléoside est fixé covalement par un bras de couplage, par l'intermédiaire de son extrémité 3'OH. L'extrémité 5'OH du nucléoside est protégée par un groupe di-p-méthoxytrityl acido-labile. Cette approche, utilisant la chimie des phosphites triesters, et dans laquelle des désoxynucléosides 3'phosphoramidites sont utilisés comme synthons est appelée la méthode des phosphoramidites. Cette approche est la plus utilisée actuellement et présente l'avantage d'être entièrement automatique.

Une autre approche utilisée pour la synthèse d'oligonucléotides est celle de la chimie des phosphonates. Cette approche commence par la condensation d'un désoxynucléoside 3'-H-phosphonate avec un déoxynucléoside couplé à un support de verre ou de silice. Des cycles de condensation successifs conduisent à la synthèse d'oligonucléotides H-phosphonates. Ces oligonucléotides sont oxydés en une étape pour donner les phosphodiesters.

En utilisant l'une ou l'autre de ces techniques, ou tout autre procédure séquentielle permettant la synthèse de chaînes polynucléotidiques de séquence déterminée à l'avance, on obtient des oligonucléotides présentant la structure de départ désirée.

Des synthèses détaillées d'oligonucléotides phosphorothioates diesters ont été décrites, par exemple, dans J. Am. Chem. Soc. 106: 6077-6079 (1984) et Nucleic Acids Res. 14: 5399-5407 (1986).

Les oligonucléotides peuvent être utilisés à titre de réactifs ou principes actifs dans des compositions diagnostiques, cosmétologiques ou pharmacologiques diverses à des concentrations variables et avec les excipients appropriés selon les applications.

D'autres caractéristiques et avantages de la présente invention apparaîtront à la lumière des exemples qui vont suivre.

La Figure 1 représente les mécanismes de décomposition intracellulaire des groupements bioréversibles.

### I - Exemples 1 à 3: synthèse des dinucléosides pliosphorothioztes triesters 4a, 4b et 4c et évaluation de leur stabilité en milieu biologique

### I-1 - synthèse des composes 4a 4b et 4c

### Conditions générales:

Les chromatographies sur couche mince (C.C.M.) sont réalisées sur plaque de silice Merck 60 F₂₅₄®. Les produits sont détectés à la lampe U.V. (254 nm) et révélés par chauffage après pulvérisation d'acide sulfurique à 10% dans l'éthanol 95°.

Les chromatographies sur colonne de gel de silice sont effectuées avec de la silice Kieselgel 60®(40µm-63µm).

Les spectres de RMN du proton sont enregistrés à température ambiante sur un appareil Brüker® AC 250. Les échantillons sont solubilisés dans le DMSO-*d*_{*6*} Les déplacements chimiques (δ) sont exprimés en ppm par rapport au signal du DMSO-*d*_{*5*} fixé à 2.49 ppm. pris comme référence interne. Les constantes de couplage sont données en Hertz. La multiplicité des signaux observés est indiquée par une lettre minuscule: m: multiplet; pq: pseudo-quartet. t: triplet; d doublet; s; singulet; sl; singulet large

On représente par N₁ le nucléoside ayant sa fonction 5'OFl libre et par N₂ le nucléoside ayant sa fonction 3'OH libre.

Les spectres de RMN du phosphore sont enregistrés à température ambiante sur un appareil Brüker® WP 200 SY avec découplage du proton. Les échantillons sont solubilisés dans CD₃CN ou dans le DMSO-*d*_{*6*}*.* Les déplacements chimiques sont exprimés en ppm par rapport à H₃PO₄ 66% pris comme référence externe.

Les spectres de masse sont effectués sur un appareil JEOL®. JMS DX 300 par la méthode d'ionisation FAB en mode positif avec différentes matrices: glycérol (G), glycérol / thioglycérol (GT) ou alcool 3-nitrobenzylique (NBA).

L'acétonitrile a été distillé après une nuit de chauffage à reflux sur hydrure de calcium et est conservé sur tamis moléculaire (4A).

Les agents d'alkylation ont été synthétisés selon des procédés décrits dans la littérature:
- iodure de pivaloyloxyméthyle: European Patent Appl N° 843080672 (EP-A - 143 601)
- bromoéthylacétylsulfure: P. Nylen, A. Olsen, *Svensk Kem. Tid.* 53, 274 (1941).
- bromométhylacétylsulfure: G.K. Farrington, A. Kumar, F.C Wedler, *Oppi briefs,* 21, 390 (1989).

Les dinucléosides phosphorothioates diesters ont été synthétisés selon des méthodes classiques décrites dans la littérature, utilisant la chimie des H-Phosphonates (J. Stawinski, M. Thelin, *Nucleosides and Nucleotides*, 9, 129 (1990). P.J. Garegg, T. Regberg, J. Stawinsky, R. Strömberg, *Nucleosides and Nucleotides*. 6. 655 (1987).

Des solutions aqueuses d'hydrogénocarbonate de triéthylammonium (TEAB) 1 M et 0.5 M. pH= 7 ont été utilisées pour neutraliser les milieux réactionnels et pour réaliser les extractions.

Avant lyophilisation. les solutions sont filtrées sur filtre Millex® HV-4 (Millipore. 0.45 µ m).

### Synthèse:

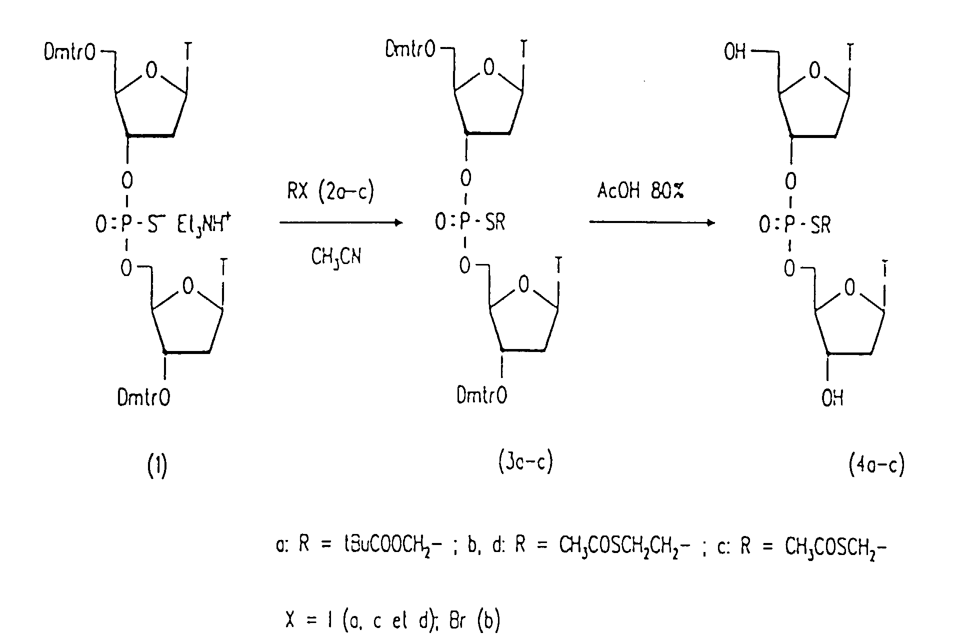

### Exemple 1

□ O-[5'-O-(4,4'-Diméthoxytrityl)thymidin-3'-yl] O-[3'-O-(4,4'-Diméthoxytrityl)thymidin-5'-yl] S-(pivaloyxyméthyl) Phosphorothioate (3a):

A une solution de dinucléoside phosphorothioate diester (1) (254 mg; 0.2 mmol.) dans l'acétonitrile anhydre (10 ml) est additionné l'iodure de pivaloyloxyméthyle (2a) (484 mg, 2 mmol.). La réaction est agitée à température ambiante durant 30 minutes, puis le milieu réactionnel est neutralisé par addition d'une solution aqueuse de TEAB 1 M (2 ml) et laissé sous agitation durant 5 minutes. Puis le mélange réactionnel est versé sur une solution aqueuse de TEAB 0,5 M (50 ml) et extrait avec du dichlorométhane (3 x 30 ml).

Les phases organiques sont rassemblées, lavées à l'eau (50 ml), séchées sur sulfate de sodium, filtrés puis évaporées à sec. Le résidu est ensuite chromatographié sur colonne de gel de silice en utilisant comme éluant un gradient de méthanol (0 à 2%) dans le dichlorométhane. Les fractions contenant (**3a**) sont rassemblées et concentrées sous pression réduite pour conduire à un résidu solide blanc (220 mg; 86%) qui est soigneusement séché pour être utilisée dans l'étape suivante.
R_{f}= 0,67 (CH₂Cl₂/MeOH: 9/1).

**□ O-(Thymidin-3'-yl) O-(Thymidin-5'-yl) S-(pivaloyloxyméthyl) Phosphorothioate (4a):**

Le dimère phosphorothioate triester totalement protégé (**3a**) est dissous dans un mélange acide acétique / eau / méthanol (8:1:1, v:v:v) (10 ml). La réaction est laissée sous agitation à température ambiante pendant 5 heures. On évapore alors le milieu réctionnel et on coévapore plusieurs fois à l'eau et au toluène. Le résidu est ensuite chromatographié sur colonne de gel de silice en utilisant comme éluant un gradient de méthanol (0 à 10%) dans le dichlorométhane. Les fractions contenant le produit (**4a**) sont rassemblées, concentrées sous pression réduite, puis le résidu est coévaporé plusieurs fois au dioxanne et lyophilisé dans le dioxanne.

On obtient (**4a**) sous forme d'une poudre blanche (98 mg, 85%).
**R**_{**f**} = 0,2 (CH₂Cl₂/MeOH: 9/1)
**MS:** FAB>0 (NBA): 677: (M+H)⁺; 551: (M-B)⁺; 424: (M-(2B+2H))⁺
**RMN** ^{**31**}**P (CD**_{**3**}**CN):** δ= 27,21 et 27,31 ppm (2 diastéréoisomères)
**RMN** ^{**1**}**H (DMSO-*d***_{***6***}**):** δ= 11,34 (sl, 2H, 2NH); 7,68 et 7,48 (2m, 2H, 2H₆); 6.21 (m, 2H, 2H_{1'}); 5,48 (d, 1H, OH_{3'}, J= 4,4 Hz); 5,41 (d, 2H, SCH₂O, J = 20 Hz); 5,26 (t, 1H, OH_{5'}, J = 5,1 Hz), 5,10 (m, 1H, H_{3'}(N₁)); 4,26 (m, 3H, H_{3'}(N₂), H_{5'} et H_{5"} (N₂₎); 4,12 (m, 1H, H_{4'}(N₁)) 3,96 (m, 1H, H_{4'}(N₂)); 3,61 (m, 2H, H_{5'} et H_{5"} (N₁)); 2,38 (m, 2H, H_{2'} et H_{2"}(N₁)); 2,14 (m, 2H, H_{2'} et H_{2"} (N₂)); 1,79 et 1,77 (2s, 2CH₃ (N₁ et N₂)); 1,13 (d, 9H, (CH₃)₃C) ppm.

### Exemple 2

□ **O-[5'-O-(4,4'-Diméthoxytrityl)thymidin-3'-yl] O-[3'-O-(4,4'-Diméthoxytrityl)thymidin-5'-yl] S-(acétylthioéthyl) Phosphorothioate (3b):**

A une solution de dinucléoside phosphorothioate diester (**1**) (50 mg; 0,0394 mmol.) dans l'acétonitrile anhydre (5 ml) est additionné le bromoéthylacétylsulfure (**2b**) (360 mg; 1,97 mmol.). Le milieu réactionnel est agité à température ambiante durant 5 jours, puis il est neutralisé par addition d'une solution aqueuse de TEAB 1 M (1 ml) et laissé sous agitation pendant 5 minutes. Le mélange réactionnel est ensuite versé sur une solution aqueuse de TEAB 0,5 M (25 ml) et extrait avec du dichlorométhane (3 x 15 ml). Les phases organiques sont rassemblées, lavées à l'eau (25 ml), séchées sur sulfate de sodium, filtrées puis évaporées à sec. Le résidu est ensuite chromatographié sur colonne de gel de silice en utilisant comme éluant un gradient de méthanol (0 à 4%) dans le dichlorométhane. On obtient (**3b**) sous forme d'un solide blanc (27 mg, 55%).
**R**_{**f**} = 0,75 (CH₂Cl₂/MeOH: 9/1).

□ **O-(Thymidin-3'-yl) O-(Thymidin-5'-yl) S-(acétylthioéthyl) Phosphorothioate (4b):**

Le dinucléoside phosphorothioate triester totalement protégé (**3b**) est dissous dans un mélange acide acétique/eau/méthanol (8:1:1, v:v:v) (5 ml) et laissé sous agitation toute une nuit. Le lendemain, on évapore le milieu réactionnel et on coévapore plusieurs fois à l'eau puis au toluène. Le résidu solide est ensuite chromatographié sur colonne de gel de silice en utilisant comme éluant un gradient de méthanol (0 à 10%) dans le dichlorométhane. Les fractions contenant le dinucléoside phosphorothioate triester détritylé (**4b**) sont rassemblées, évaporées sous pression réduite, puis le résidu est coévaporé au dioxanne et lyophilisé dans le dioxanne. On obtient (**4b**) sous forme d'une poudre blanche (12 mg, 86%).
**R**_{**f**} = 0,35 (CH₂Cl₂/MeOH:9/1)
**MS:** FAB>0 (GT): 665: (M+H)⁺
**RMN** ^{**31**}**P (DMSO-*d***_{***6***}**)**: δ = 28,09 et 28,22 ppm
**RMN** ^{**1**}**H (DMSO-*d***_{***6***}**):** δ = 11,34 (2s, 2H, 2NH); 7,68 et 7,48 (2d, 2H, 2H₆); 6,20 (t, 2H, 2H_{1'}); 5,48 (d, 1H, OH_{3'}), 5,26 (pq, 1H, OH_{5'}), 5,09 (m, 1H, H_{3'} (N₁)) 4,26 (m, 3H, H_{3'}(N₂), H_{5'} et H_{5"}(N2)); 4,10 (m, 1H, H_{4'}(N₁)); 3,95 (m, 1H, H_{4'}(N₂)); 3,61 (m, 2H, H_{5'} et H_{5"}(N₁)); 3,13 et 3,02 (2m, 4H, SCH₂CH₂S); 2,29 (m, 2H, H_{2'} et H_{2"} (N₁)); 2,33 (d, 3H, CH₃CO); 2,13 (m, 2H, H_{2'} et H_{2"}(N₂)), 1,77 et 1.78 (2s, 6H, 2CH₃ (N₁ et N₂)) ppm.

### Exemple 3

□ **O-[5'-O-(4,4'-Diméthoxytrityl)thymidin-3'-yl] O-[3'-O-(4,4'-Diméthoxytrityl)thymidin-5'-yl] S-(acétylthiométhyl) Phosphorothioate (3c)**:

A une solution de dinucléoside phosphorothioate diester **(1)** (58 mg; 0,0457 mmol.) dans l'acétonitrile anhydre (5 ml) est additionné l'iodométhylacétylsulfure (98 mg; 0,457 mmol.). La réaction est laissée sous agitation toute une nuit à température ambiante, puis le milieu réactionnel est neutralisé par addition d'une solution aqueuse de TEAB 1 M (1 ml) et laissé sous agitation durant quelques minutes. Puis le mélange réactionnel est versé sur une solution aqueuse de TEAB 0,5 M (25 ml) et extrait avec du dichlorométhane (3 × 15 ml). Les phases organiques sont rassemblées, lavées à l'eau (25 ml), séchées sur sulfate de sodium, filtrées puis évaporées à sec. Le résisu est ensuite chromatographié sur colonne de gel de silice en utilisant comme éluant un gradient de méthanol (0 à 3%) dans le dichlorométhane. On obtient (**3c**) sous forme d'un solide blanc (46 mg, 80%)
**R**_{**f**}= 0,55 (CH₂Cl₂/MeOH : 9/1).

**□ O-(thymidin-3'-yl) O-(thymidin-5'-yl) S-(acétylthiométhyl) Phosphorothioate (4c)**:

Le dinucléoside phosphorothioate totalement protégé (3c) est dissous dans un mélange acide acétique / eau / méthanol (8:1:1, v:v:v) (5 ml) et laissé sous agitation à température ambiante toute une nuit, Le lendemain, on évapore le milieu réactionnel et on coévapore plusieurs fois à l'eau et au toluène, Le résidu est ensuite chromatographié sur colonne de gel de silice en utilisant comme éluant un gradient de méthanol (0 à 10%), Les fractions contenant le dinucléoside phosphorothioate triester détritylé **(4c)** sont rassemblées et concentrées sous pression réduite. Le résidu est coévaporé au dioxanne puis lyophilisé dans le dioxanne, On obtient (**4c**) sous forme d'une poudre blanche (20 mg, 85%).
**R**_{**f**} = 0,62 (CH₂Cl₂/MeOH : 8/2)
**MS**: FAB>0 (GT): 651 (M+H)⁺
**RMN** ^{**31**}**P (CD**_{**3**}**CN):** δ = 27,22 et 27,44 ppm (2 diastéréoisomères),
RMN ¹H (DMSO-*d*_{*6*}): δ = : 11,34 (d, 2H, 2 NH); 7,70 et 7,49 (2d, 2H, 2H₆); 6.22 (m, 2H, 2H_{1'}); 5,48 (d, 1H, OH_{3'}); 5,37 (m, 1H, OH_{5'}); 5,08 (m, 1H, H₃, (N₁)); 4,29 (m. 5H, H_{3'}(N₂), H_{5'} et H_{5"}(N₂), SCH₂S); 4,13 (m, 1H, H_{4'}(N₁)); 3,97 (m, 1H, H_{4'} (N₂)); 3,62 (m, 2H, H_{5'} et H_{5"} (N₁)), 2,39 (m. 5H, H_{2'} et H_{2"} (N₁), CH₃CO), 2,15 (m. 2H, H_{2'}et H_{2"} (N₂)); 1,78 (s. 6H, 2CH₃ (N₁ et N₂)).

### I-2 - Etudes de stabilité des dimères phosphorothioates triesters 4a, 4b et 4c

### Conditions générales:

### • Méthode

La stabilité des dimères (4a), (4b) et (4c) dans différents milieux biologiques a été étudiée selon une technique C.L.H.P. mise au point au laboratoire (A. Pompon, I, Lefebvre, J.-L. Imbach, *Biochemical Pharmacology.* 43, 1769 (1992)), qui ne nécessite aucune manipulation préalable de l'échantillon et permet son injection directe: une précolonne, qui permet d'éliminer les protéines, remplie d'un nouveau matériau I.S.R.P. (Internal Surface Reverse Phase) est associée à une colonne haute résolution (phase inverse) permettant l'analyse chromatographique.

### • Matériel:

Le chromatographe (Waters-Millipore) est composé de :
- un programmateur M 680
- deux pompes M 510
- un injecteur automatique WISP 712
- un détecteur U.V. à barrette de diodes M 990
- un microordinateur NEC APC IV
- une imprimante Waters 990
- une vanne 6 voies modèle 7010 (Rhéodyne).

Les colonnes sont fournies par la S.F.C.C.-Shandon:
- Précolonne I.S.R.P. (Ultrabiosep®C 18, 10 µm, 4,6 mm × 10 mm)
- Colonne analytique phase inverse (Nucléosil® C18, 5µm, 4,6 mm × 100 mm)

La colonne analytique est thermostatée à 30°C,

L'exploitation des résultats se fait sur le logiciel EUREKA.

### • Produits chimiques:

L'eau distillée est purifiée sur un système MilliQ® (Waters-Millipore),

L'acétonitrile est de qualité H.P.L.C.-lointain U.V. (Fisons).

L'acétate d'ammonium est de qualité "pour analyses" (MERCK).

Les milieux de culture sont composés de 90% de RPMI 1640 et de 10% de sérum de veau foetal inactivé par la chaleur (GIBCO),

Les extraits cellulaires ont été aimablement fournis par M^{elle} ,A.-M. Aubertin (Université de Strasbourg I). Ils sont préparés de la façon suivante: des cellules CEM en croissance exponentielle sont séparées du milieu de culture par centrifugation (10⁴g. 4 min. 4°). Le résidu (environ 100 µl, 5.10⁷ cellules) est mis en solution dans 2 ml de tampon (Tris-HCl 140 nM. pH=7,4) et soniqué. Le lysat est centrifugé (10⁵g. 1h, 4°) pour éliminer les membranes, les organites et la chromatine.

Deux types d'éluants ont été utilisés pour la C.L.H.P.:
- éluant A: Tampon acétate d'ammonium 0,1 M. pH = 5,9.
- éluant B: Tampon acétate d'ammonium 0,1 M. acétonitrile 50%. pH = 5.9.

### • Préparation des échantillons:

On prépare une solution 10⁻² M du composé à étudier dans le DMSO.

Cette solution est diluée avec de l'eau pour conduire à une solution mère de concentration 5.10⁻⁴ M.

### a) - étude de stabilité en milieu de culture:

100 µl de solution mère du composé à étudier sont ajoutés à 900 µl de milieu de culture préalablement filtrés sur filtre stérile Millex® GV (Millipore, 0.22 µm). Après mélange, des fractions (100 µl) sont réparties dans des tubes Eppendorf stériles. Ces tubes sont placés dans une étuve à 37°C et prélévés en fonction de l'évolution cinétique. Les échantillons sont immédiatement analysés par C.L.H.P. (volume injecté. 8O µl) ou conservés à -25^{°}C en vue d'une analyse ultérieure.

### b) - étude de stabilité en extrait cellulaire:

100 µl de solution mère du composé à étudier sont ajoutés à 900 µl d'extrait cellulaire préalablement filtrés sur filtre stérile Millex® GV (Millipore, 0.22 µm). Après mélange, des fractions (100 µl) sont réparties dans des tubes Eppendort. Ces tubes sont placés dans une étuve à 37°C et prélèves en fonction de l'évolution cinétique. Les échantillons sont immédiatement analysés par C.L.H.P..

### Résultats:

Les résultats concernant les études de stabilité des trois dimères (4a), (4b) et (4c) sont rassemblées dans le tableau ci-dessous:
C₀ = 5.10⁻⁵ M
t_{1/2} = temps au bout duquel la moitié du produit de départ s'est décomposée
PO⁻ = dinucléoside phosphodiester
PS⁻ = dinucléoside phosphorothioate diester
% PO⁻, % PS⁻ = fractions molaires de dinucléoside phosphodiester et de dinucléoside phosphorothioate diester, exprimées par rapport à la quantité initiale de dinucléoside phosphorothioate triester.

Le pourcentage de PS⁻ correspond à la quantité de dinucléoside phosphorothioate diester formé après décomposition complète du dinucléoside phosphorothioate triester de départ (en considérant que le dinucléoside phosphorothioate diester est stable dans les conditions utilisées et s'accumule).

Le pourcentage de PO⁻ est calculé par la différence (100 - % PS⁻ formé) car le dinucléoside phosphodiester se décompose rapidement dés sa formation.

| | RPMI + 10% sérum | | | extrait cellulaire | | |
|---|---|---|---|---|---|---|
| | t_{1/2} | % PO⁻ | % PS⁻ | t_{1/2} | % PO⁻ | % PS⁻ |
| (4a) | 6h | 80 | 20 | 40 min | / | 100 |
| (4b) | 7h | 50 | 50 | 10 min | / | 100 |
| (4c) | 1h | 20 | 80 | 5-10 min | / | 100 |

II découle de l'étude de stabilité des composés 4a-c présentée ci-dessus que ceux-ci sont rapidement et sélectivement transformés en extrait cellulaire en leur phosphorothioate diester correspondant, ce qui est en accord avec l'hypothèse originelle.

### II - Exemples 4 à 6: synthèse d'oligonucléotides contenant des liaisons phosphorothioates triesters par alkylation post-synthèse de liaisons phosphorothioates diesters. Evaluation de leur stabilité en milieux biologiques

### II-1 - synthèse des oligonucléotides 5c, 6b et 7b contenant des liaisons phosphorothioates triesters

### Conditions générales:

Les oligonucléotides contenant des liaisons phosphorothioates diesters ont été synthétisés en phase solide sur un synthétiseur d'ADN Applied Biosystems modèle 381 A. sur des colonnes de support correspondant à une µmole de nucléoside gréffé.

La purification et l'analyse des oligonucléotides 5c, 6b et 7b ont été effectuées par C.L.H.P. en utilisant un système chromatographique composé de matériel Waters-Millipore:
- un programmateur M 680
- un ordinateur NEC APC IV
- une imprimante Waters 990
- deux pompes M 510
- un injecteur U₆K
- un détecteur U.V. à barrettes de diode

Les colonnes et les précolonnes sont fournies par la SFCC-Shandon:
- dans le cas de C.L.H.P. préparative, on a utilisé une colonne Nucléosil® C₁₈ N 525 (10mm × 250 mm) de granulométrie 5 µm protégée par une précolonne Nucléosil® C₁₈ PSF-25 de granulométrie 5 µm.
- dans le cas de C.L.H.P. analytique, on a utilisé une colonne Nucléosil® C₁₈ N125 (4,6 mm x 150 mm) de granulométrie 5 µm protégée par une précolonne Nucléosil C₁₈ PSF-25 de granulométrie 5 µm.

Après purification, les oligonucléotides sont filtrés sur filtre Millex® HV-13 (Millipore. 0,45 µm) et soumis à plusieurs lyophilisations successives dans l'eau.

Le suivi des réactions d'alkylation des oligonucléotides et la purification des oligonucléotides alkylés 5c, 6b et 7b ont été effectués par C.L.H.P. sur un chromatographe Waters-Millipore composé de :
- un programmateur 600 E
- un système de pompage 600
- un injecteur manuel U₆K
- un détecteur U.V. 486
- un micro-ordinateur Powermate SX Plus

Le suivi des réactions d'alkylation a été réalisé sur une colonne Nucléosil® C₁₈ 5µm (4,6mm × 150 mm) protégée par une précolonne Nucléosil® C₁₈ 5µm (S.F.C.C.-Shandon).

La purification des oligonucléotides 5c, 6b et 7b a été effectuée sur une cartouche RADIAL-PAK® C₁₈ 10µm pour support de colonne RCM (8 × 10), protégée par une précolonne GUARD-PAK Resolve C₁₈ (Waters-Millipore).

Le dessalage des échantillons a été réalisé sur une cartouche C₁₈ SEP-PAK® (Waters-Millipore).

Avant lyophilisation, les solutions sont filtrées sur filtre Millex® HV-13 (Millipore, 0.45 µm)

### II-1-1 - Exemple 4: synthèse du dodécamére chimérique 5c contenant une fenêtre centrale phosphorothioate triester et des flancs méthylphosphonates.

**a) - synthèse d'un dodécamère contenant une fenêtre centrale phosphorothioate diester et des flancs méthylphsophonates.** 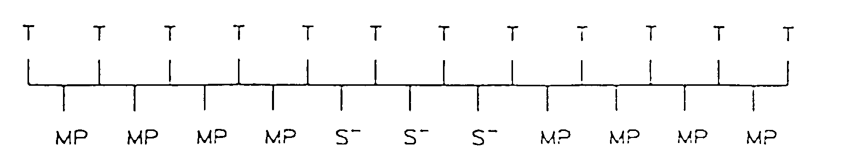
   On a utilisé le cycle standard d'élongation proposé pour la synthése des oligonucléotides_{.} en utilisant pour les flancs le synthon méthylphosphonamidite et le mélange I₂/THF/ eau / pyridine comme agent d'oxydation, et pour la partie centrale (3 cycles d'incorporations) le synthon cyanoéthylphosphoramidite et le réactif de Beaucage comme agent de sulfuration (R.P. Iyer, W. Egan, J.B. Regan, S.L. Beaucage. J. *Am. Chem. Soc.,* **112**, 1253 (1990)).
   Le décrochage de l'oligonucléotide du support et sa déprotection ont été réalisés selon le procédé classiquement décrit pour les méthylphosphonates (P.S. Miller, M.P. Reddy, A. Murakami, K.R. Blake, S.B. Lin, C.H. Agris, *Biochemistry,* **25**, 5092 (1986).) en utilisant une solution d'éthylènediamine dans l'éthanol absolu (1:1, v:v).
   La synthèse sur l'échelle d'une micromole nous a conduit à un brut de synthèse de 75 unités d'absorbance à 260 nm (unités A₂₆₀).
   Après purification par C.L.H.P. préparative, nous avons obtenu 37 unités A₂₆₀ d'oligonucléotide chimérique d'une pureté spectrophotométrique de 100% (déterminée par analyse C.L.H.P.).
**b) - Alkylation des liaisons phosphorothioates diesters.** 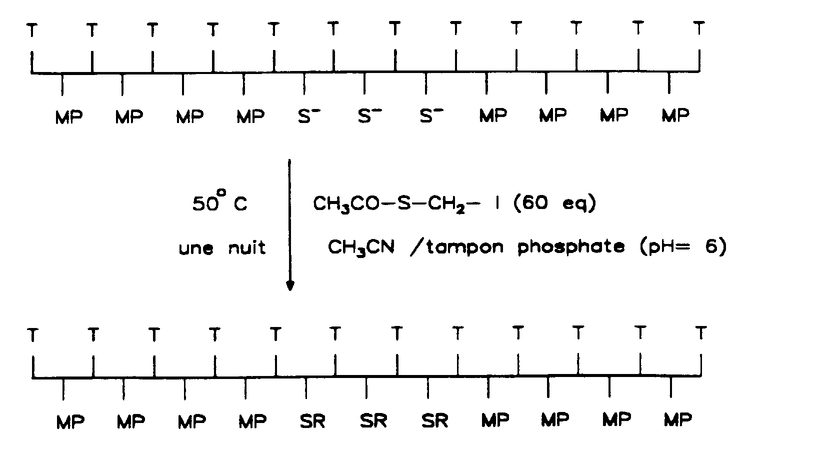
Dans un eppendorf, on introduit successivement:
- 120 µl d'acétonitrile
- 135 µl de tampon phosphate 20 mM (pH = 6,15)
- 15 µl d'iodométhylacétylsulfure en solution 0,92 M dans l'acétonitrile
- 30 µl de solution 7,66 mM d'oligonucléotide dans l'eau (soit environ 22 unités A₂₆₀)

L'eppendorf est placé dans un bain à sec prélablement thermostaté à 50°C.

Des prélèvements de 5 µl sont effectués à différents temps de réaction et sont analysés par C.L.H.P. afin d'estimer l'avancement de la réaction.

Après 16 heures de réaction, on ne détecte plus la présence de l'oligonucléotide de départ (tᵣ= 12,99 min) et l'oligonucléotide totalement alkylé (tᵣ= 17,53 min) est purifié par C.L.H.P..

L'oligonucléotide est ensuite dessalé, avant d'être lyophilisé dans un mélange eau / dioxanne (50:50, v:v) et analysé à nouveau par C.L.H.P..

On a obtenu 10 unités A₂₆₀ d'oligonucléotide totalement alkylé d'une pureté spectrophotométrique de 97% (déterminée par C.L.H.P.).

### II-1-2 - exemple 5: synthèse du dodécamère 6b entièrement phosphorothioate triester.

**a) - synthèse d'un dodécamère phosphorothioate diester d(C**_{**P(S)**}**A**_{**P(S)**}**C**_{**P(S)**}**C**_{**P(S)**}**C**_{**P(S)**}**A**_{**P(S)**}**A**_{**P(S)**}**T**_{**P(S)**}**T**_{**P(S)**}**C**_{**P(S)**}**T**_{**P(S)**}**G)**
   On a utilisé le cycle standard d'élongation proposé pour la synthèse d'oligonucléotides en utilisant les synthons cyanoéthylphosphoramidites des quatre bases convenablement protégés et en remplaçant l'étape d'oxydation par 12 / THF / eau / pyridine par une étape de sulfuration avec le réactif de Beaucage.
   Le décrochage de l'oligonucléotide du support et sa déprotection ont été réalisées par un traitement à l'ammoniaque concentré (32%) pendant une nuit à 55°C.
**b) - alkylation des liaisons phosphorothioates diesters.**
   A une solution 6,4 mM d'oligonucléotide phosphorothioate diester, d(C_{P(S)}A_{P(S)}C_{P(S)}C_{P(S)}C_{P(S)}A_{P(S)}A_{P(S)}T_{P(S)}T_{P(S)}C_{P(S)}T_{P(S)}G) dans de l'eau (42 µl) on ajoute successivement du tampon phosphate (pH= 6,15, 123 µl), de l'acétonitrile (120 µl) et une solution 1 M d'iodoéthylacétylsulfure (**2d**) (55 µl). Le mélange est maintenu à 50°C pendant 48 heures. Après évaporation, le résidu est purifié par C.L.H.P.. La fraction contenant le dodécamère phosphorothioate entièrement alkylé 6b est dessalé et le résidu d'évaporation repris dans un mélande eau / dioxanne (50:50, v:v, 1 ml) est lyophilisé.
   Rendement 35%. La référence (2d) se rapporte à la nomenclature utilisée dans le schéma de synthèse du paragraphe I-1.

### II-1-3 - exemple 6: synthèse du dodécamère chimérique 7b contenant une fenêtre centrale constituée de β-nucléosides phosphorothioates triesters et des flancs constitués d'α-nucléosides phosphates diesters.

**a) - synthèse du dodécamère contenant une fenêtre β-phosphorothioate diester et des flancs** α**-phosphates diesters d[α-(TCTT)3'→ 3' β-(T**_{**P(S)**}**T**_{**P(S)**}**C**_{**P(S)**}**C)5'→ 5'α-(CTCT)]**
   On a utilisé le cycle standard d'élongation proposé pour la synthèse des oligonucléotides en utilisant pour les flancs les synthons cyanoéthylphosphoramidites d'anomène α convenablement protégés sur les bases et le mélange I₂ / THF / eau / pyridine comme agent d'oxydation, et pour la partie centrale les synthons cyanoéthylphosphoramidites d'anomérie β convenablement protégés sur les bases et le réactif de Beaucage comme agent de sulfuration.
**b) - alkylation des liaisons phosphorothioates diesters.**
   A une solution 11 mM d'oligonucléotide d[α-(TCTT)3'→3'β-(T_{P(S)}T_{P(S)}C_{P(S)}C)5'→5' α-(CTCT)] dans de l'eau (20 µl), on ajoute successivement du tampon phosphate (pH= 6,15, 145 µl) et une solution 1 M d'iodoéthylacétylsulfure (**2d**) (55 µl). Le mélange est maintenu à 50°C pendant 48 heures. Après évaporation, le résidu est purifié par C.L.H.P..
   La fraction contenant le dodécanucléotide (7d) alkylé sur les trois liaisons phosphorothioates centrales est dessalée puis lyophilisée. Rendement 47%.

### II-2 - Etude de stabilité des oligonucléotides chimériques 5c, 6b et 7b dans des milieux biologiques.

### Conditions générales:

Les conditions générales sont les mêmes que celles utilisées pour les études de stabilité des dimères phosphorothioates triesters, en ce qui concerne la méthode, le matériel et les produits chimiques

### • Préparation des échantillons:

on prépare une solution mère d'oligonucléotide dans le dioxanne (10 unités A₂₆₀ d'oligonucléotide dans 200 µl de dioxanne).
**a) - étude de stabilité en milieu de culture:**
   On prélève 30 µl de solution mère d'oligonucléotide (soit environ 1,5 unités A₂₆₀), que l'on ajoute à 1470 µl de milieu de culture préalablement filtré sur filtre stérile Millex GV (Millipore, 0,22 µm). Après mélange, des fractions (100 µl) sont réparties dans des tubes Eppendorf stériles. Ces tubes sont placés dans une étuve à 37°C et prélevés en fonction de l'évolution cinétique. Les échantillons sont immédiatement analysés par C.L.H.P (volume injecté 80 µl) ou conservés à -25°C en vue d'une analyse ultérieure.
**b) - étude de stabilité en extrait cellulaire:**
   10 µl de solution mère d'oligonucléotide sont ajoutés à 990 µl d'extrait cellulaire préalablement filtrés sur filtre stérile Millex GV (Millipore, 0,22 µm). Après mélange, des fractions (100 µl) sont réparties dans des tubes Eppendorf stériles, placées dans une étuve à 37°C, prélevées à des temps différents et immédiatement analysées par C.L.H.P..

### Résultats:

Les résultats concernant les études de stabilité des trois oligonucléotides 5c, 6b et 7b sont rassemblés dans le tableau ci-dessous

| | **RPMI + 10% sérum** | | **extrait cellulaire** |
|---|---|---|---|
| **oligonucléotide étudié** | **t**_{**1/2**} **de disparition de l'oligonucléotide de départ** | **t**_{**1/2**} **de disparition de l'oligonucléotide de départ** | **t**_{**1/2**} **de formation de l'oligonucléotide totalement déprotégé** |
| **5c** | **40 min** | **< 5 min** | **20 min** |
| **6b** | **55 min** | **< 2 min** | **25 min** |
| **7b** | **35 min** | **< 2 min** | **20 min** |

L'ensemble des données concernant les composes **5c, 6b** et **7b** montre sans ambiguité qu'il est possible de protéger sélectivement par des groupements bioréversibles des fonctions internucléotidiques phosphorothioates. De plus, il est bien confirmé en extrait cellulaire que de tels oligonucléotides phosphorothioates triesters sont rapidement deprotégés.

**La Figure 1** représente le mécanisme de décomposition des groupements bioréversibles selon l'invention.

## Revendications

1. Oligonucléotide phosphorothioate triester caractérisé en ce qu'il comporte des enchaînements internucléotidiques qui présentent une liaison P-S- protégée par un groupement (X) bioréversible dans les milieux intracellulaires.

2. Oligonucléotide selon la revendication 1 caractérisé en ce qu'il répond à la formule générale suivante 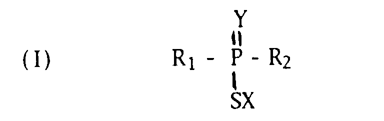 dans laquelle :
- Y représente O ou S
- R₁ et R₂ sont respectivement un résidu 3'- O et 5'- O d'un nucléoside ou d'un oligonucléotide dont l'enchaînement internucléotidique est naturel ou modifié, notamment dont l'enchaînement est du type phosphorothioate triester de formule 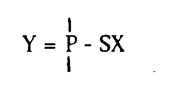
- X est un radical -(CH₂)ₙ -Y¹- W où
. Y¹ représente S ou O
. n varie de 1 à 6
. W représente :
- soit SU avec U qui représente un radical alkyle, aryle, ou osidique éventuellement substitués,
- soit 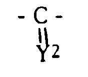 Z avec Y² qui représente O ou S, et
Z qui représente un radical alkyle, aryle, ou osidique éventuellement substitués.

3. Oligonucléotide selon la revendication 2 caractérisé en ce que X représente -(CH₂)ₙ - S - S - U ou

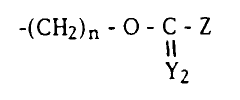

4. Oligonucléotide selon la revendication ou 3 caractérisé en ce que X représente 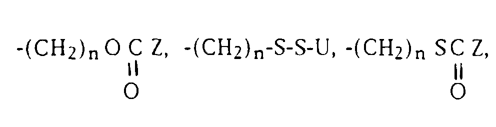 avec n = 1 ou 2.

5. Oligonucléotide selon l'une des revendications 2 à 4 caractérisé en ce que U et Z sont un alkyl inférieur éventuellement substitué par un ou des groupe(s) choisi(s) parmi OH, SH, ou NH₂.

6. Oligonucléotide selon la revendication 5 caractérisé en ce que X représente -(CH₂)ₙ - S - S - (CH₂)_{n¹} - X¹ avec n et n¹ qui représentent un entier de 1 à 4, de préférence 2 et X¹ représente H, OH, SH ou NH₂, ou X représente -(CH₂)ₙ - Y¹ - CO - Z, avec Z = CH₃ ou tBu.

7. Oligonucléotide selon l'une des revendications 1 à 6 caractérisé en ce qu'il est constitué d'un oligomère chimérique comportant une séquence centrale d'ADN ou d'ARN dont les enchaînements internucléotidiques comportent une liaison 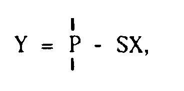 cette séquence centrale étant flanquée en 5' et 3' de séquences d'ADN ou ARN modifiées de sorte qu'elles résistent aux nucléases et/ou stabilisent l'hybridation avec un brin complémentaire.

8. Oligonucléotide selon l'une des revendications 2 à 7 caractérisé en ce que R₁ et R2 ont des séquences aux extrémités 5' et 3' respectivement, électriquement neutres, et qui résistent à la dégradation par les exonucléases.

9. Oligonucléotide selon la revendication 8 caractérisé en ce que les enchaînements internucléotidiques aux extrémités 5' et 3' respectivement de R₁ et R₂ sont du type méthyl-phosphonate.

10. Oligonucléotide selon l'une des revendications 1 à 7 caractérisé en ce qu'il est constitué d'un oligomère chimérique comportant une séquence centrale d'ADN ou d'ARN d'anomérie β, dont les enchaînements internucléotidiques sont du type 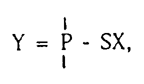 cette séquence centrale étant flanquée en 5' et 3' de séquences d'ADN ou d'ARN d'anomérie alpha.

11. Oligonucléotide selon la revendication 10 caractérisé en ce qu'il est constitué d'un oligomère chimérique contenant une séquence centrale d'encadrement de β-nucléosides phosphorothioates triesters à enchaînement internucléotidique de type 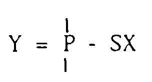 encadrée par des flancs constitués d'enchaînements d'alpha nucléosides phosphates diesters.

12. Procédé de préparation d'un oligonucléotide selon l'une des revendications 1 à 11 caractérisé en ce qu'on effectue une réaction de substitution nucléophile par l'atome de soufre de la liaison -P-S⁻ d'un oligonucléotide phosphorothiate diester sur un agent alkylant XL, conduisant ainsi à la formation d'enchaînements internucléotidiques phosphorothiates triesters 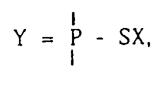 L étant un groupe partant tel halogène, ester, tosyl, et X étant un groupement bioréversible tel que défini dans les revendications 1 à 11.

## Patentansprüche

1. Phosphorothioattriester-Oligonucleotid, dadurch gekennzeichnet, daß es Internucleotid-Kettenverknüpfungen enthält, die eine P-S⁻Bindung aufweisen, die in den intrazellulären Medien durch eine bioreversible Gruppierung (X) geschützt ist.

2. Oligonucleotid nach Anspruch 1, dadurch gekennzeichnet, daß es die folgende allgemeine Formel hat 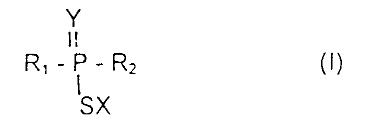 worin bedeuten:
- Y O oder S₁,
- R₁ und R₂ jeweils einen 3'-O- oder 5'-O-Rest eines Nucleosids oder eines Oligonucleotids mit natürlicher oder modifizierter Internucleotid-Kettenverknüpfung, wobei die Kettenverknüpfung insbesondere eine solche vom Phosphorothioattriester-Typ der Formel ist 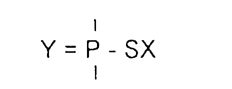
- X einen Rest -(CH₂)ₙ-Y¹-W, worin Y¹ für S oder O steht, n von 1 bis 6 variiert und W entweder für SU, worin U einen gegebenenfalls substituierten Alkyl-, Aryl- oder Osid-Rest darstellt, oder für 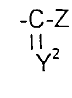 steht, worin Y² O oder S und Z einen gegebenenfalls substituierten Alkyl-, Aryl- oder Osid-Rest darstellen.

3. Oligonucleotid nach Anspruch 2, dadurch gekennzeichnet, daß X steht für -(CH₂)ₙ-S-S-U oder

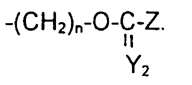

4. Oligonucleotid nach Anspruch 2 oder 3, dadurch gekennzeichnet, daß X steht für 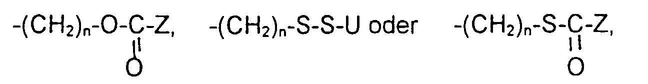 worin n = 1 oder 2.

5. Oligonucleotid nach einem der Ansprüche 2 bis 4, dadurch gekennzeichnet, daß U und Z stehen für niederes Alkyl, das gegebenenfalls durch eine oder mehrere der Gruppen, ausgewählt aus OH, SH und NH₂, substituiert ist.

6. Oligonucleotid nach Anspruch 5, dadurch gekennzeichnet, daß X steht für -(CH₂)ₙ-S-S-(CH₂)_{n¹}-X¹, worin n und n¹ eine ganze Zahl von 1 bis 4, vorzugsweise die Zahl 2, und X¹ H, OH, SH oder NH₂ bedeuten, oder X steht für -(CH₂)ₙ-Y¹-CO-Z worin Z CH₃ oder t-Bu darstellt.

7. Oligonucleotid nach einem der Ansprüche 1 bis 6, dadurch gekennzeichnet, daß es aufgebaut ist aus einem Chimären-Oligomer, das eine zentrale DNA- oder RNA-Sequenz aufweist, deren Internucleotid-Kettenverknüpfungen eine Y=P-SX-Bindung aufweisen, wobei diese zentrale Sequenz in 5'-und 3'-Stellung von modifizierten DNA- oder RNA-Sequenzen flankiert ist, so daß sie gegen Nucleasen resistent sind und/oder die Hybridisierung mit einem komplementären Strang stabilisieren.

8. Oligonucleotid nach einem der Ansprüche 2 bis 7, dadurch gekennzeichnet, daß R₁ und R₂ an den jeweils elektrisch neutralen 5'- und 3'-Enden Sequenzen aufweisen, die gegen Abbau durch Exonucleasen beständig sind.

9. Oligonucleotid nach Anspruch 8, dadurch gekennzeichnet, daß die Internucleotid-Kettenverknüpfungen an den jeweiligen 5'- und 3'-Enden von R₁ und R₂ solche vom Methylphosphonat-Typ sind.

10. Oligonucleotid nach einem der Ansprüche 1 bis 7, dadurch gekennzeichnet, daß es aus einem Chimären-Oligomer aufgebaut ist, das eine zentrale β-Anomerie-DNA- oder RNA-Sequenz aufweist, deren Internucleotid-Kettenverknüpfungen solche vom 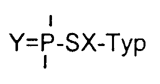 sind, wobei diese zentrale Sequenz in 5'- und 3'-Stellung von α-Anomerie-DNA- oder -RNA-Sequenzen flankiert ist.

11. Oligonucleotid nach Anspruch 10, dadurch gekennzeichnet, daß es aus einem Chimären-Oligomer aufgebaut ist, das eine zentrale Einrahmungs-Sequenz von Phosphorothioat-triester-β-Nucleosiden mit einer Internucleotid-Kettenverknüpfung vom 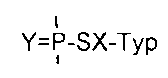 enthält, die durch Flanken eingerahmt ist, die aus Phosphatdiester-α-Nucleosid-Kettenverknüpfungen bestehen.

12. Verfahren zur Herstellung eines Oligonucleotids nach einem der Ansprüche 1 bis 11, dadurch gekennzeichnet, daß man eine nucleophile Substitutionsreaktion mit dem Schwefelatom der -P-S⁻Bindung eines Phosphorothioatdiester-Oligonucleotids an einem Alkylierungsmittel YL durchführt, die so zur Bildung von Phosphorothioat-triester-Internucleotid-Kettenverknüpfungen 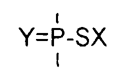 führt, wobei L für eine austretende Gruppe, wie Halogen, Ester und Tosyl, und X für eine bioreversible Gruppierung, wie sie in den Ansprüchen 1 bis 11 definiert ist, stehen.

## Claims

1. Phosphorothioate triester oligonucleotide, characterized in that it comprises internucleotide linkages which have a P-S⁻ bond protected by a group (X) which is bioreversible in intracellular media.

2. Oligonucleotide according to Claim 1, characterized in that it corresponds to the following general formula 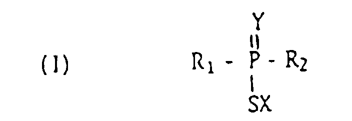 in which:
- Y is O or S
- R₁ and R₂ are respectively a residue in the 3'-O and 5'-O positions of a nucleoside or of an oligonucleotide, the internucleotide linkage of which is natural or modified, the linkage of which is especially of the phosphorothioate triester type of formula 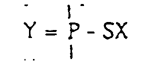
- X is a -(CH₂)ₙ-Y¹-W radical where
· Y¹ is S or O
· n varies from 1 to 6
· W is:
- either SU with U being an optionally substituted alkyl, aryl or oside radical,
- or 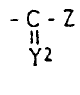 with Y² being O or S, and Z being an optionally substituted alkyl, aryl or oside radical.

3. Oligonucleotide according to Claim 2, characterized in that X is
(CH₂)ₙ - S - S - U
ou

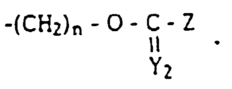

4. Oligonucleotide according to Claim 2 or 3, characterized in that X is: 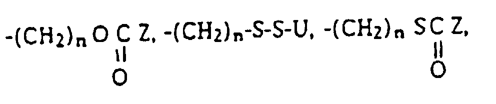 where n = 1 or 2.

5. Oligonucleotide according to one of Claims 2 to 4, characterized in that U and Z are a lower alkyl, optionally substituted by one or more groups chosen from amongst OH, SH and NH₂.

6. Oligonucleotide according to Claim 5, characterized in that X is (CH₂)ₙ-S-S-(CH₂) _{n¹}-X¹ with n and n¹ being an integer from 1 to 4, preferably 2, and X¹ is H, OH, SH or NH₂, or X is -(CH₂)ₙ-Y¹-CO-Z, where Z = CH₃ or tBu.

7. Oligonucleotide according to one of Claims 1 to 6, characterized in that it is formed of a chimeric oligomer comprising a central DNA or RNA sequence, the internucleotide linkages of which comprise a 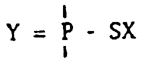 bond, this central sequence being flanked in the 5' and 3' positions by DNA or RNA sequences modified so that they are resistant to nucleases and/or stabilize hybridization with a complementary strand.

8. Oligonucleotide according to one of Claims 2 to 7, characterized in that R₁ and R₂ have electrically neutral sequences at the 5' and 3' ends respectively, which are resistant to degradation by exonucleases.

9. Oligonucleotide according to Claim 8, characterized in that the internucleotide linkages at the 5' and 3' ends respectively of R₁ and R₂ are of the methylphosphonate type.

10. Oligonucleotide according to one of Claims 1 to 7, characterized in that it is formed from a chimeric oligomer comprising a β-anomeric central DNA or RNA sequence, the internucleotide linkages of which are of the 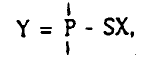 type, this central sequence being flanked in the 5' and 3' positions by α-anomeric DNA or RNA sequences.

11. Oligonucleotide according to Claim 10, characterized in that it is formed from a chimeric oligomer containing a surrounding central sequence of phosphorothioate triester β-nucleosides with internucleotide linkage of the 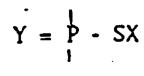 type surrounded by flanks formed from phosphate diester alpha-nucleoside linkages.

12. Method of preparation of an oligonucleotide according to one of Claims 1 to 11, characterized in that a nucleophilic substitution reaction by the sulphur atom of the -P-S⁻ bond of a phosphorothioate diester oligonucleotide is carried out on an alkylating agent XL, thus leading to the formation of 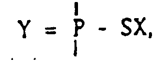 phosphorothioate triester internucleotide linkages, L being a leaving group such as halogen, ester or tosyl, and X being a bioreversible group such as defined in Claims 1 to 11.
